# EUROPEAN PATENT APPLICATION

(11) **EP 2 808 812 A1**
(43) Date of publication of application: **03.12.2014**
(21) Application number: 12866680.7
(22) Date of filing: 09.08.2012
(51) Int. Cl.: G06F 19/00, G06F 17/50

(54) **ANALYSIS DEVICE AND SIMULATION METHOD**

(30) Priority: 26.01.2012 JP 2012013721
(71) Applicant: Sumitomo Heavy Industries, Ltd., Tokyo 141-6025 (JP)
(72) Inventor: OHNISHI, Yoshitaka, Yokosuka-shi Kanagawa 237-8555 (JP)
(74) Representative: Louis Pöhlau Lohrentz
(86) International application number: PCT/JP2012/005069
(87) International publication number: WO 2013/111204

(57) **Abstract**

An analysis device includes a unit cell acquisition unit configured to acquire a unit cell under a period boundary condition reflecting symmetry of an object to be analyzed, a unit particle system acquisition unit configured to acquire a unit particle system including a plurality of unit particles in the unit cell acquired by the unit cell acquisition unit, and a numerical computation unit configured to numerically compute a governing equation, which governs the motion of each unit particle of the unit particle system acquired by the unit particle system acquisition unit, under the period boundary condition. The numerical computation unit moves the boundary of the unit cell to simulate the movement of the object to be analyzed during computation.

## Description

### Incorporation by Reference

Priority is claimed to Japanese Patent Application No. 2012-013721, filed January 26, 2012, and International Patent Application No. PCT/JP2012/005069, the entire content of each of which is incorporated herein by reference.

### BACKGROUND

### Technical Field

The present invention relates to an analysis device which analyzes a particle system, and a simulation method.

### Description of the Related Art

In the related art, as a method of investigating a phenomenon of a general material science using a computer based on classical mechanics, quantum mechanics, or the like, a molecular dynamics method (hereinafter, referred to as an MD method, and for example, see the related art) is known. In a simulation based on the MD method, the motion of a particle can be physically handled more exactly compared to a finite element method (for example, see the related art) or the like. However, in the MD method, if the number of particles increases, since the amount of calculation increases rapidly, only a comparatively small number of particles are handled in practical use. Accordingly, in the related art, there are many cases where the MD method is primarily used for the purpose, in which the shape of the object to be analyzed is less involved, such as prediction of physical properties of materials.

When calculating the property of a bulk using the MD method, in general, a period boundary condition is applied. Under the period boundary condition, a substantially cubic unit cell is defined, and the same space as the unit cell is repeatedly assumed in three orthogonal directions of x, y, and z (this is referred to as a replica cell). In addition, it is assumed that the exact same motion of an atom as the motion of an atom in the unit cell occurs in the respective replica cells. For example, if an atom in the unit cell exits out of the boundary surface of the unit cell, the same kind of atom having the same speed as the exited atom enters the corresponding position of the unit cell from an adjacent replica cell through the opposite boundary surface of the unit cell simultaneously. Accordingly, it is possible to perform a simulation while maintaining the number of atoms in the system. When obtaining a force applied from an atom outside the unit cell to an atom in the unit cell, since the atom position of the replica cell is known, the force can be calculated using the atom position.

If the period boundary condition is imposed, a calculation region is arranged infinitely, whereby it is possible to reproduce a bulk state with no surface.

In recent years, a renormalized molecular dynamics method (hereinafter, referred to as an RMD method) which is developed from the MD method to handle a system on macro scale is suggested (for example, see the related art). With the RMD method, an object to be analyzed spreads to a real structure, such as a gear, a motor, or a beam.

### SUMMARY

According to an embodiment of the invention, there is provided an analysis device including a cell acquisition unit configured to acquire a unit cell under a period boundary condition reflecting symmetry of an object to be analyzed, a particle system acquisition unit configured to acquire a system including a plurality of particles in the unit cell acquired by the cell acquisition unit, and a numerical computation unit configured to numerically compute a governing equation, which governs the motion of each particle of the system acquired by the particle system acquisition unit, under the period boundary condition. The numerical computation unit moves the boundary of the unit cell to simulate the movement of the object to be analyzed during computation.

According to another embodiment of the invention, there is provided a simulation method in which, when analyzing an object having rotational symmetry using a renormalized molecular dynamics method, a period boundary condition reflecting rotational symmetry of the object is set.

### BRIEF DESCRIPTION OF THE DRAWINGS

Fig. 1 is a schematic view showing an example of a unit cell.
Fig. 2 is a block diagram showing the functions and configuration of an analysis device according to an embodiment.
Fig. 3 is a data structural diagram showing an example of a unit particle information retaining unit of Fig. 2.
Fig. 4 is a flowchart showing an example of a sequence of processing in the analysis device of Fig. 1.
Figs. 5A and 5B are schematic views showing a unit particle system corresponding to a workpiece and a work particle model corresponding to a work.
Figs. 6A to 6D are side views showing a mode of temporal development of the unit particle system.
Figs. 7A to 7D are side views showing a mode of temporal development of the unit particle system.

### DETAILED DESCRIPTION

In general, a real structure has a shape which is not isotropic. Accordingly, the above-described instinctive period boundary condition for use in the MD method is unsuitable as a boundary condition which is applied when simulating a real structure using the RMD method.

It is desirable to provide an analysis technique capable of reducing a calculation load in a simulation using a particle method.

According to the embodiment of the invention, it is possible to use the unit cell corresponding to the moving object to be analyzed.

It is to be noted that any arbitrary combination of the above-described structural components or rearrangement of the structural components and the expressions of the invention among a device, a method, a system, a computer program, a recording medium having a computer program recorded thereon, and the like are also effective as the embodiments of the invention.

According to the embodiment of the invention, it is possible to reduce a calculation load in a simulation using a particle method.

Hereinafter, the same or similar structural components, members, and processing shown in the respective drawings are represented by the same reference numerals, and overlapping description will be appropriately omitted.

In an analysis device according to an embodiment, when analyzing an object having rotational symmetry using an RMD method, a period boundary condition reflecting rotational symmetry of the object is set. With this, since it is possible to reduce the number of particles to be computed using rotational symmetry of the object, it is possible to reduce a calculation load.

In this embodiment, although a case where a particle system is analyzed using the RMD method will be described, it is obvious to those skilled in the art of contact with the specification that the technical idea of this embodiment can be applied to a case where a particle system is analyzed using other particle methods, such as an MD method with no renormalization, a distinct element method (DEM), smoothed particle hydrodynamics (SPH), and moving particle semi-implicit (MPS).

The user generates a unit particle system and a unit cell in the following exemplary procedure as an initial condition for numerical computation in the analysis device according to the embodiment.

The user selects a real object having rotational symmetry, such as a gear or a disk, as an object to be analyzed. The user generates an object region having a similar shape to the shape of the object to be analyzed in a three-dimensional virtual space using the analysis device or other computation devices. The user disposes particles in the MD method, that is, a plurality of particles corresponding to a real-world atom or molecule in the generated object region. In an example, the number of particles to be disposed is about an Avogadro number.

Hereinafter, a case where all particles are set to have the same quality or similar quality, and a potential energy function is a pair potential and set to have the same shape regardless of particles will be described. However, it is obvious to those skilled in the art of contact with the specification that the technical idea of this embodiment can be applied to other cases.

The user applies a conversion rule in the RMD method to a system including a plurality of particles disposed. In an example, the number of particles is reduced to about millions by millions of times of renormalization. The user defines a unit cell under a period boundary condition reflecting rotational symmetry of the object to be analyzed for the renormalized system. The user defines a system including a plurality of particles in the defined unit cell as a unit particle system. The unit particle system is a part of the renormalized system.

A unit cell may be defined for the system including a plurality of particles disposed, and a conversion rule may be applied to a system including particles in the defined unit cell.

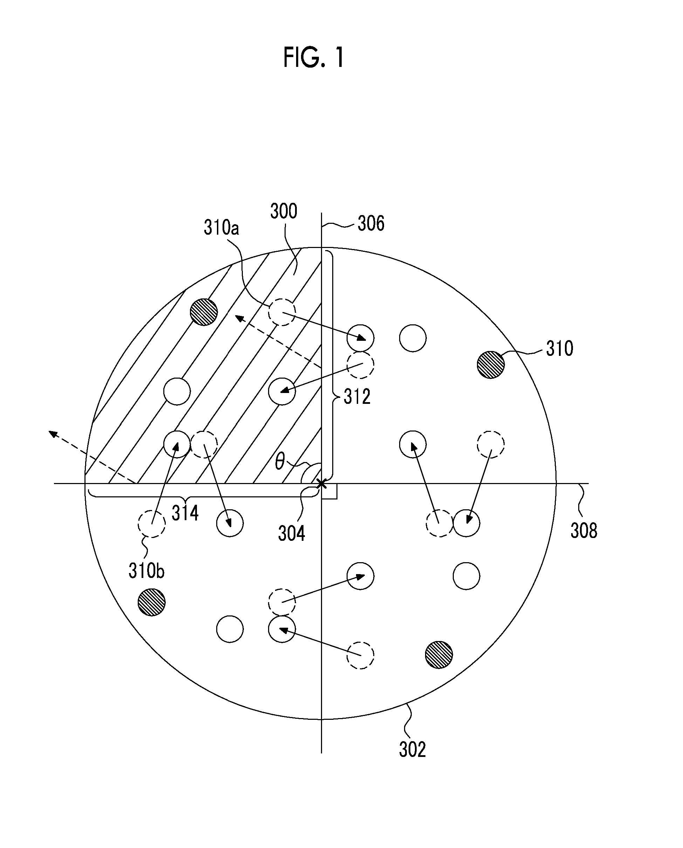

Fig. 1 is a schematic view showing an example of a unit cell 300. In this example, an object to be analyzed has a uniform disk shape. In general, since symmetry of a system is maintained before and after renormalization by the RMD method, an object region (hereinafter, referred to as a renormalized object region) 302 after renormalization also has a uniform disk shape. Particles 310 of the renormalized system are disposed in the renormalized object region 302.

The user defines a first plane 306 which includes a rotation axis 304 (an axis extending in a direction orthogonal to the paper surface of Fig. 1) as a reference of rotational symmetry of the renormalized object region 302, and a second plane 308 which includes the rotation axis 304 and is orthogonal to the first plane 306. Since the renormalized object region 302 is divided into four regions by the first plane 306 and the second plane 308, the user selects one region of the divided regions as the unit cell 300. The user defines a system including a plurality of particles 310 in the selected unit cell 300 as a unit particle system. The unit particle system is a subset of the renormalized system. With this meaning, it can be said that the definition of the unit particle system is an operation to cut the unit particle system from the renormalized system.

The unit cell 300 is a fan-shaped region having a predetermined thickness, and a central angle θ thereof is 90 degrees. The surface of the unit cell 300 includes a first boundary surface 312 which is a part of the first plane 306, and a second boundary surface 314 which is a part of the second plane 308, and the unit cell 300 is a region which is sandwiched between these boundary surfaces. The first boundary surface 312 and the second boundary surface 314 are planes which extend from the rotation axis 304 to the boundary of the renormalized object region 302 outward in a radial direction.

Although the user defines the second boundary surface 314 as a part of the second plane 308, alternatively, a surface which is obtained by rotating the first boundary surface 312 by 90 degrees (an angle of 1/4 of 360 degrees) in a counterclockwise direction around the rotation axis 304 may be defined as the second boundary surface 314.

During numerical computation in the analysis device, when a particle 310a exits from a certain position on the first boundary surface 312 to the outside at a certain speed, a corresponding particle 310b enters the unit cell 300 from a corresponding position on the second boundary surface 314 at a corresponding speed. The same applies to a case where a particle runs outside through the second boundary surface 314.

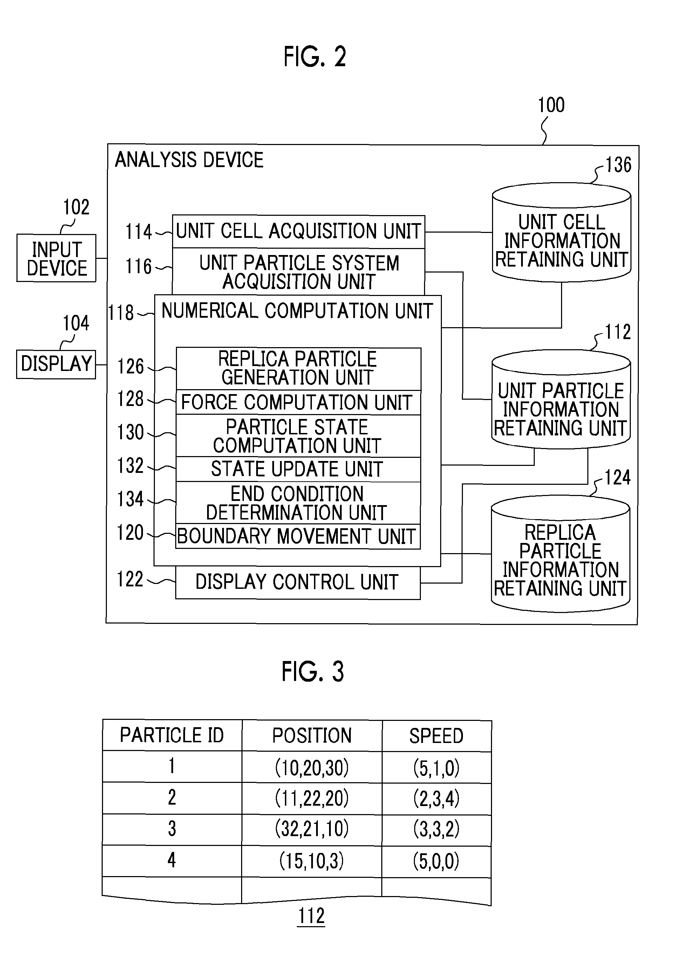

Fig. 2 is a block diagram showing the functions and configuration of an analysis device 100 according to the embodiment. Although respective blocks shown in the drawing can be realized by hardware, for example, an element, such as a central processing unit (CPU) of a computer, or a mechanical device, or can be realized by software, for example, a computer program or the like, functional blocks which are realized by collaboration of hardware and software are drawn. Accordingly, it is understood by those skilled in the art of contact with the specification that these functional blocks can be realized by combination of hardware and software in various ways.

The analysis device 100 is connected to an input device 102 and a display 104. The input device 102 may be a device which receives an input related to processing executed on the analysis device 100 from the user, for example, a keyboard or a mouse. The input device 102 may be configured to receive an input from a network, such as Internet, or a recording medium, such as a compact disk (CD) or a digital versatile disk (DVD).

The analysis device 100 includes a unit cell acquisition unit 114, a unit particle system acquisition unit 116, a numerical computation unit 118, a display control unit 122, a unit particle information retaining unit 112, a unit cell information retaining unit 136, and a replica particle information retaining unit 124.

The unit cell acquisition unit 114 acquires the unit cell 300 defined by the user through the input device 102. The unit cell acquisition unit 114 registers information, which expresses the acquired unit cell 300 in a virtual space, in the unit cell information retaining unit 136. Information which expresses the unit cell 300 in a virtual space is, for example, functions representing a plane, a curved surface, a line, or a curve, or a combination thereof, and in particular, includes information which expresses the first boundary surface 312 and the second boundary surface 314.

The unit particle system acquisition unit 116 acquires the unit particle system defined by the user through the input device 102. Hereinafter, it is assumed that the unit particle system includes Q (where Q is a natural number) unit particles. The unit particle system acquisition unit 116 registers a particle ID for specifying a unit particle of the unit particle system, the position vector (hereinafter, simply referred to as position) of the unit particle, and the speed vector (hereinafter, simply referred to as speed) of the unit particle in the unit particle information retaining unit 112 in association with one another.

The numerical computation unit 118 numerically computes a governing equation, which governs the motion of each unit particle of the unit particle system acquired by the unit particle system acquisition unit 116, under the period boundary condition. In particular, the numerical computation unit 118 performs repetitive computation according to an equation of motion of discretized particles. In the computation of the numerical computation unit 118, a condition that the object to be analyzed moves in the radial direction is imposed. The numerical computation unit 118 moves the first boundary surface 312 and the second boundary surface 314 in the same direction during the computation (see a broken-line arrow of Fig. 1). The direction of movement corresponds to the direction of movement of the object to be analyzed. The numerical computation unit 118 updates the position and speed of each unit particle of the unit particle system based on the computation result.

The numerical computation unit 118 includes a replica particle generation unit 126, a force computation unit 128, a particle state computation unit 130, a state update unit 132, an end condition determination unit 134, and a boundary movement unit 120.

The replica particle generation unit 126 refers to the unit particle information retaining unit 112, and rotates each unit particle of the unit particle system around the rotation axis 304 by θ = 90 degrees, 2θ = 180 degrees, and 3θ = 270 degrees and duplicates the unit particle. The duplicated particle is called a replica particle. The replica particle generation unit 126 registers a particle ID for specifying a replica particle, the position of the replica particle, and the speed of the replica particle in the replica particle information retaining unit 124 in association with one another. In Fig. 1, colored circles represent a unit particle and three replica particles corresponding to the unit particle.

The force computation unit 128 refers to the unit particle information retaining unit 112 and the replica particle information retaining unit 124, and for each unit particle of the unit particle system, computes a force applied to the unit particle based on the distance between particles. At this time, the force computation unit 128 may compute the distance between each unit particle and all replica particles to compute the force applied to the unit particle. In particular, the force computation unit 128 may compute the distance between each unit particle and replica particles, which are a replica of the unit particle to compute the force applied to the unit particle by the replica particles.

For an i-th (where 1 ≤ i ≤ Q) unit particle of the unit particle system, the force computation unit 128 decides unit particles or replica particles (hereinafter, referred to as near particle) which are at a distance from the i-th unit particle smaller than a predetermined cutoff distance. For each near particle, the force computation unit 128 computes the force applied to the i-th unit particle by the near particle based on the potential energy function between the near particle and the i-th unit particle and the distance between the near particle and the i-th unit particle. In particular, the force computation unit 128 calculates the force from the value of the gradient of the potential energy function in the value of the distance between the near particle and the i-th unit particle. The force computation unit 128 adds the force applied to the i-th unit particle by the near particle for all near particles to calculate the force applied to the i-th unit particle.

The particle state computation unit 130 refers to the unit particle information retaining unit 112, and applies the force computed by the force computation unit 128 to the equation of motion of discretized particles for each unit particle to compute at least one of the position and speed of the unit particle. In this embodiment, the particle state computation unit 130 computes both the position and speed of the unit particle.

The particle state computation unit 130 computes the speed of the unit particle from the equation of motion of discretized particles including the force computed by the force computation unit 128. The particle state computation unit 130 substitutes the force computed by the force computation unit 128 in the equation of motion of particles discretized using a predetermined minute time interval Δt based on a predetermined numerical analysis method, such as a leapfrog method or an Euler method, for the i-th unit particle, thereby computing the speed of the unit particle. In the computation, the speed of the unit particle computed in the previous repetitive computation cycle is used.

The particle state computation unit 130 calculates the position of the unit particle based on the computed speed of the unit particle. The particle state computation unit 130 applies the computed speed of the unit particle to a relationship expression of position and speed of discretized particles using the time interval Δt based on a predetermined numerical analysis method for the i-th unit particle, thereby computing the position of the unit particle. In the computation, the position of the unit particle computed in the previous repetitive computation cycle is used.

The state update unit 132 updates the position and speed of each unit particle retained in the unit particle information retaining unit 112 to the position and speed computed by the particle state computation unit 130. The state update unit 132 refers information of the first boundary surface 312 and information of the second boundary surface 314 retained in the unit cell information retaining unit 136, and for each unit particle, performs determination about whether or not the unit particle exits from the first boundary surface 312 or the second boundary surface 314 to the outside of the unit cell 300 as a result of updating the position. The state update unit 132 rotates a unit particle, which is determined to exit to the outside of the unit cell 300, around the rotation axis 304 toward the unit cell 300 along with the direction of the speed. The absolute value of the angle of the rotation is θ = 90 degrees. That is, the state update unit 132 rotates the position vector and the speed vector of a unit particle exited from the first boundary surface 312 to the outside of the unit cell 300 in Fig. 1 by 90 degrees in a counterclockwise direction, and rotates the position vector and speed vector of a unit particle exited from the second boundary surface 314 to the outside of the unit cell 300 by 90 degrees in a clockwise direction.

The state update unit 132 may add the position and speed computed by the particle state computation unit 130 in the unit particle information retaining unit 112, instead of updating the unit particle information retaining unit 112.

The end condition determination unit 134 performs determination about whether or not to end repetitive computation in the numerical computation unit 118. An end condition for ending repetitive computation is, for example, that repetitive computation is performed a predetermined number of times, an end instruction is received from the outside, or the system reaches a normal state. When the end condition is satisfied, the end condition determination unit 134 ends repetitive computation in the numerical computation unit 118.

When the end condition is not satisfied in the end condition determination unit 134, the boundary movement unit 120 moves the first boundary surface 312 and the second boundary surface 314 in the same direction. The amount of single movement in this case is set to a unit movement distance which is a distance obtained by multiplying a set movement speed by the time interval Δt. Specifically, the boundary movement unit 120 registers a boundary surface obtained by moving the first boundary surface 312 in parallel at the unit movement distance in the direction of movement in the unit cell information retaining unit 136 as a new first boundary surface 312. The same applies to the second boundary surface 314. Processing when a unit particle exits to the outside of the unit cell 300 with the movement of the boundary surface is equivalent to processing in the state update unit 132. After the boundary movement processing in the boundary movement unit 120, the process returns to the replica particle generation unit 126.

The display control unit 122 displays a mode of temporal development of the unit particle system or a state at a certain time on the display 104 based on the position and speed of each unit particle of the unit particle system retained in the unit particle information retaining unit 112.

Fig. 3 is a data structural diagram showing an example of the unit particle information retaining unit 112. The unit particle information retaining unit 112 retains a particle ID, the position of the particle, and the speed of the particle in association with one another. The replica particle information retaining unit 124 has the same data structure as shown in Fig. 3.

In the above-described embodiment, an example of the retaining unit is a hard disk or a memory. It is understood by those skilled in the art of contact with the specification that the respective units can be realized by a CPU (not shown), a module of an installed application program, a module of a system program, a memory which temporarily stores the contents of data read from a hard disk, or the like based on the description of the specification.

The operation of the analysis device 100 having the above-described configuration will be described.

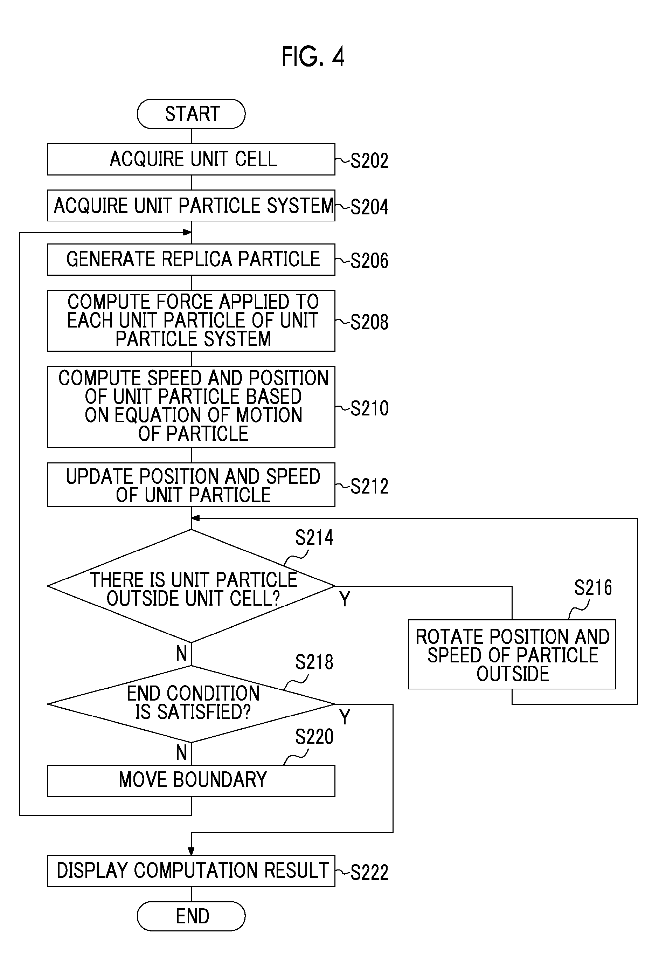

Fig. 4 is a flowchart showing an example of a sequence of processing in the analysis device 100. The unit cell acquisition unit 114 acquires information of the unit cell defined by the user (S202). The unit particle system acquisition unit 116 acquires information of the unit particle system defined by the user (S204). The replica particle generation unit 126 generates replica particles corresponding to each unit particle (S206). The force computation unit 128 computes the force applied to each unit particle (S208). The particle state computation unit 130 computes the position and speed of each unit particle based on the equation of motion of particles (S210). The state update unit 132 updates the position and speed of each unit particle (S212). The state update unit 132 performs determination about whether or not there is a unit particle outside the unit cell (S214). When there is a unit particle (Y in S214), the state update unit 132 rotates the position and speed of the exited particle (S216), and the process returns to Step S214. When there is no unit particle (N in S214), the end condition determination unit 134 performs determination about whether or not the end condition is satisfied (S218). When the end condition is not satisfied (N in S218), the boundary movement unit 120 moves the boundary surfaces of the unit cell (S220), and the process returns to Step S206. When the end condition is satisfied (Y in S218), the display control unit 122 displays the computation result on the display 104 (S222).

According to the analysis device 100 of this embodiment, when simulating the object to be analyzed moving with rotational symmetry using the RMD method, the period boundary condition reflecting rotational symmetry is set. The first boundary surface 312 and the second boundary surface 314 of the unit cell 300 move in the same direction to correspond to the movement of the object to be analyzed under the period boundary condition during numerical computation. Accordingly, even when the object to be analyzed moves, the period boundary condition can be applied in the same manner as when the object to be analyzed is stationary. That is, a particle system corresponding to the entire object to be analyzed in a moving state does not need to be computed, and only the particles in the unit cell 300 are to be computed, whereby it is possible to simulate the behavior of the object to be analyzed in the moving state. Therefore, it is possible to reduce the number of particles which are handled in numerical computation, and to reduce a calculation load.

The inventors have simulated how a workpiece is deformed by spinning using the analysis device 100 as a preliminary experiment.

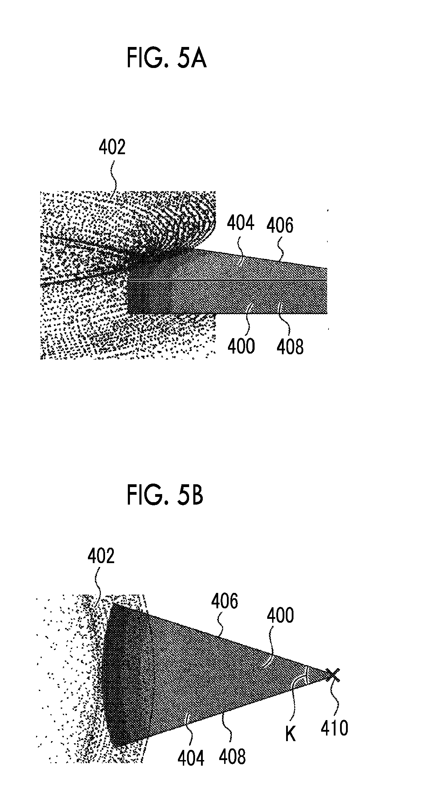

Figs. 5A and 5B are schematic views showing a unit particle system 400 corresponding to a workpiece and a work particle model 402 corresponding to a work. Fig. 5A is a perspective view, and Fig. 5B is a top view. The workpiece has a disk shape, and the central angle K of the unit cell 404 is 36.5 degrees. The workpiece moves toward a work at a predetermined speed. In response to this, for a first boundary surface 406 and a second boundary surface 408 of the unit cell 404, the same speed as the direction from the rotation axis 410 toward the center of the work particle model 402 is set.

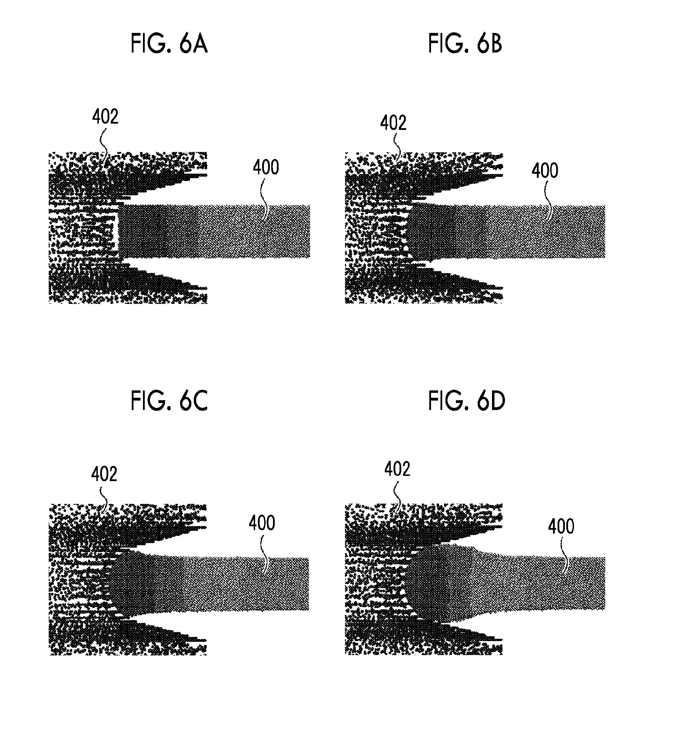

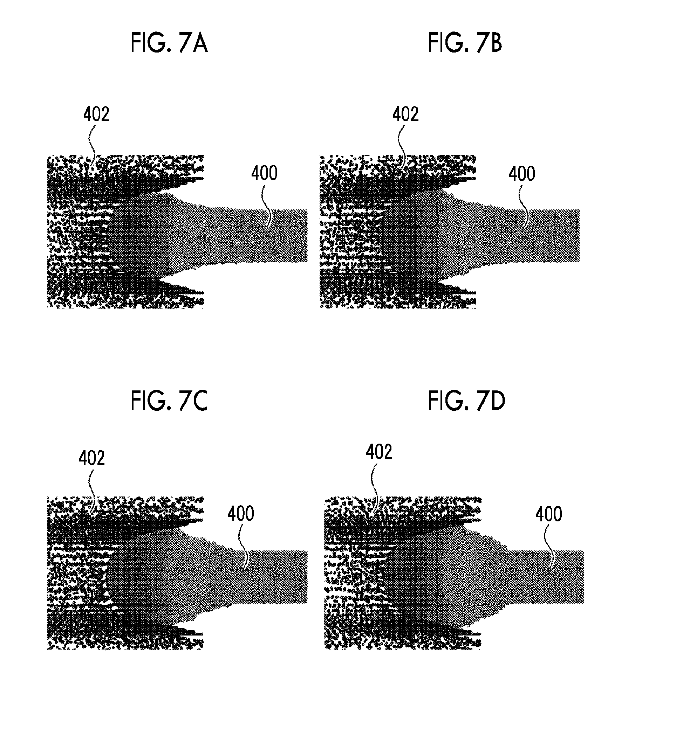

Figs. 6A to 6D are side views showing a mode of temporal development of the unit particle system 400. Figs. 7A to 7D are side views showing a mode of temporal development of the unit particle system 400. Figs. 6A, 6B, 6C, 6D, 7A, 7B, 7C, and 7D are time-sequentially arranged in this order. According to the comparison and examination of the inventors, the simulation result describes that plastic deformation in spinning is satisfactory. In this way, if the analysis device 100 of this embodiment is used, it becomes possible to more accurately understand a phenomenon in an object to be analyzed while moving.

A cylindrical period boundary condition is often used in a finite element method or the like. However, in the MD method, in general, since an object region is a region on nano scale, there is less need for devising a boundary condition with an object to be analyzed on macro scale in mind. The inventors have found out that, if the MD method is developed to the RMD method, since the shape of an object to be analyzed can be one of important factors in a simulation, it is advantageous to use a period boundary condition reflecting symmetry of the object to be analyzed in the RMD method. In particular, when simulating an object to be analyzed having rotational symmetry using the RMD method, a cylindrical period boundary condition is applied, whereby it is possible to reduce a calculation load in numerical computation and to improve precision of the simulation result.

The configuration and operation of the analysis device 100 according to the embodiment have been described. It is understood by those skilled in the art that the embodiment is just for illustration, and various modifications may be made to a combination of structural components or processing and still fall within the scope of the invention.

In the embodiment, although a case where an object to be analyzed has rotational symmetry has been described, the invention is not limited thereto, and the technical idea of this embodiment can be applied to a case where an object to be analyzed has different symmetry, such as mirror-image symmetry.

In the embodiment, although a case where a three-dimensional virtual space is used has been described, the invention is not limited thereto, and there is no limit to the dimension of the virtual space, such as a two-dimensional virtual space.

In the embodiment, although a case where the renormalized object region 302 has continuous rotational symmetry, and one of the four divided regions of the renormalized object region 302 is defined as the unit cell 300 has been described, the invention is not limited thereto, and for example, when N is a natural number equal to or greater than 2, one of N divided regions of the renormalized object region 302 may be defined as a unit cell. In this case, a period boundary condition is also realized by continuous rotational symmetry of the renormalized object region 302. That is, if θ' is 360 degrees/N, the replica particle generation unit may rotate each unit particle of the unit particle system around the rotation axis 304 by an angle of θ', 2θ', ..., and (N-1)θ' and may duplicate the unit particle to generate replica particles.

In the embodiment, although a case where both the first boundary surface 312 and the second boundary surface 314 are planes has been described, the invention is not limited thereto, and for example, even if both the first boundary surface and the secondboundary surface are curved surfaces, it should suffice that these shapes correspond to each other. That is, when an object to be analyzed has continuous rotational symmetry, a unit cell may be a region sandwiched between a first boundary surface which extends in a radial direction from a rotation axis and a second boundary surface which is obtained by rotating the first boundary surface at an angle of 1/M (where M is a natural number equal to or greater than 2) of 360 degrees around the rotation axis.

In the embodiment, although a case where an object to be analyzed has continuous rotational symmetry has been described, the invention is not limited thereto, and for example, the technical idea of this embodiment may be applied to a case where an object to be analyzed has discrete rotational symmetry. For example, when an object to be analyzed has P-fold symmetry (where P is a natural number equal to or greater than 2), a unit cell may be defined as a region sandwiched between a first boundary surface which extends in a radial direction from a rotation axis and a second boundary surface which is obtained by rotating the first boundary surface at an angle of 1/P of 360 degrees around the rotation axis.

In the embodiment, although a case where both the position and speed of the particle are computed in the numerical computation unit 118 has been described, the invention is not limited thereto. For example, as a method for numerical analysis, like a Verlet method, a method in which, when computing the position of the particle, the position of the particle may be computed directly from the force applied to the particle, and the speed of the particle may not be computed explicitly is known, and the technical idea of this embodiment may be applied to this method.

According to the invention, it is possible to reduce a calculation load in a simulation using a particle method.

It should be understood that the invention is not limited to the above-described embodiment, but may be modified into various forms on the basis of the spirit of the invention. Additionally, the modifications are included in the scope of the invention.

## Claims

1. An analysis device comprising:
a cell acquisition unit configured to acquire a unit cell under a period boundary condition reflecting symmetry of an obj ect to be analyzed;
a particle system acquisition configured to acquire a system including a plurality of particles in the unit cell acquired by the cell acquisition unit; and
a numerical computation unit configured to numerically compute a governing equation, which governs the motion of each particle of the system acquired by the particle system acquisition unit, under the period boundary condition,
wherein the numerical computation unit moves the boundary of the unit cell to simulate the movement of the object to be analyzed during computation.

2. The analysis device according to claim 1,
wherein the object to be analyzed has rotational symmetry,
the cell acquisition unit acquires, as the unit cell, a region sandwiched between a first boundary surface, which extends from a rotation axis in a radial direction, and a second boundary surface, which is obtained by rotating the first boundary surface around the rotation axis at an angle of 1/N (where N is a natural number equal to or greater than 2) of 360 degrees, and
the numerical computation unit moves the first boundary surface and the second boundary surface in the same direction during computation.

3. The analysis device according to claim 1,
wherein the system acquired by the particle system acquisition unit is a renormalized system using a renormalized molecular dynamics method.

4. A simulation method,
wherein, when analyzing an object having rotational symmetry using a renormalized molecular dynamics method, a period boundary condition reflecting rotational symmetry of the object is set.

5. A computer program which causes a computer to realize:
a function of acquiring a unit cell under a period boundary condition reflecting symmetry of an object to be analyzed;
a function of acquiring a system including a plurality of particles in the acquired unit cell;
a function of numerically computing a governing equation, which governs the motion of each particle of the acquired system, under the period boundary condition; and
a function of moving the boundary of the unit cell to simulate the movement of the object to be analyzed during computation.
